# EUROPEAN PATENT APPLICATION

(11) **EP 2 289 554 A1**
(43) Date of publication of application: **02.03.2011**
(21) Application number: 09167010.9
(22) Date of filing: 31.07.2009
(51) Int. Cl.: A61K 45/06

(54) **Compositions comprising thalidomide and artemisinin for the treatment of cancer**

(71) Applicant: Dafra Pharma N.V., 2300 Turnhout (BE)
(72) Inventor: Jansen, Frans Herwig, 2360, OUD-TURNHOUT (BE)
(74) Representative: van Kooij, Adriaan

(57) **Abstract**

The present invention relates to pharmaceutical compositions for the treatment of cancer and especially for the treatment of hematological malignancies such as multiple myeloma. Specifically, the present invention relates to pharmaceutical composition for the treatment of cancer comprising: thalidomide, or a derivative thereof, or salts or solvates thereof; one or more artemisinin compounds, or salts or solvates thereof; and one or more pharmaceutically acceptable carriers and/or excipients.

## Description

The present invention relates to pharmaceutical compositions for the treatment of cancer, and especially for the treatment of hematological malignancies such as multiple myeloma.

Cancer, or malignant neoplasm, is a class of diseases in which a group of cells display uncontrolled growth (division beyond the normal limits), invasion (intrusion on and destruction of adjacent tissues), and sometimes metastasis (spread to other locations in the body via lymph or blood). The malignant properties of cancers differentiate them from benign tumors, which are self-limited, and do not invade or metastasize. Most cancers form a tumor but some, like leukemia, do not.

Cancers are generally classified by the type of cell that resembles the tumor and, therefore, the tissue presumed to be the origin of the tumor. Examples of general categories include:
- Carcinoma:: Malignant tumors derived from epithelial cells. This group represents the most common cancers, including the common forms of breast, prostate, lung and colon cancer;
- Sarcoma:: Malignant tumors derived from connective tissue, or mesenchymal cells;
- Germ cell:: Tumors derived from totipotent cells. In adults most often found in the testicle and ovary; in fetuses, babies, and young children most often found on the body midline, particularly at the tip of the tailbone;
- Blastoma:: A tumor (usually malignant) which resembles an immature or embryonic tissue. Many of these tumors are most common in children; and
- Hematological:: Hematological malignancies derived from hematopoietic (blood-forming) cells.

Hematological malignancies are types of cancer that affect blood, bone marrow, and lymph nodes. As the three are intimately connected, a disease affecting one of the three will often affect the others as well. As an example, although lymphoma is technically a disease of the lymph nodes, it often spreads to the bone marrow, affecting the blood.

There are generally two normal cell lineages from which hematological malignancies derive, myeloid and lymphoid. The former normally produces granulocytes, erythrocytes, thrombocytes, macrophages and mast cells, the latter B, T, NK and plasma cells. Lymphomas, acute lymphoblastic leukemia and myeloma are from the lymphoid line, while acute and chronic myelogenous leukemia, myelodysplastic syndromes and myeloproliferative diseases are myeloid in origin.

Multiple myeloma, also designated as MM myeloma or plasma cell myeloma, is a cancer of the white blood cells or B cells. Multiple myeloma is incurable, but remissions may be induced with steroids, chemotherapy, thalidomide and stem cell transplants.

Thalidomide, chemically designated as N-phthalimido)glutarimide or 2-(2,6-dioxo-3-piperidinyl)-1H-isoindole-1,3(2H)-dione, is a racemic compound sold under the tradename THALOMID.

Of the two enantiomers of thalidomide, i.e., (S)-thalidomide and (R)-thalidomide, the (R)-thalidomide enantiomer is generally pharmaceutically effective. The (S)-thalidomide is believed to be teratogenic and the cause of birth defects. However, the (R)-thalidomide enantiomer is *in* vivo converted into the (S)-thalidomide enantiomer. Accordingly, administrating only the (R)-thalidomide enantiomer will result in serum levels of both the (R) and (S) enantiomer thereby not obviating side effects, such as a teratogenic effect, of the (S) enantiomer.

Thalidomide was first tested as a single agent for the treatment of multiple myeloma due to its anti-angiogenesis activity. Since then, many studies have shown that thalidomide in combination with dexamethasone provided increased the survival of multiple myeloma patients. The combination of thalidomide and dexamethasone, often also in combination with melphalan, is presently one of the most common regimens for patients with newly diagnosed multiple myeloma, with an improved response rate of up to 60-70%.

However, thalidomide administration may also cause side effects such as polyneuropathy, fatigue, skin rash, and venous thromboembolism (VTE), or blood clots, which could lead to stroke or myocardial infarction. After a systematic review of VTE associated with thalidomide in multiple myeloma patients, it was reported that when thalidomide was administered without prophylaxis, VTE rates reached as high as 26%.

Amongst others due to these side effects, derivatives of thalidomide have been developed such as lenalidomide, chemically designated as 3-(4-amino-1-oxo 1,3-dihydro-2H-isoindol-2-yl) piperidine-2,6-dione and marketed as revlimid, and bortezomib, chemically designated as [(1R)-3-methyl-1-[[(2S)-3-phenyl-2-(pyrazine-2-carbonylamino)propanoyl]amino]butyl]boronic acid and marketed as Velcade. Another analog, actimid (CC-4047), chemically designated as 4-amino-2-(2,6-dioxopiperidin-3-yl)isoindole-1,3-dione, is in the clinical trial phase.

Dihydroartemisinin (a sesquiterpene lactone) is the active metabolite of all artemisinin derivatives and analogues such as artemisinin, artesunate, artemether, artelinic acid, artenimol or artemotil. For treatment of malaria, and especially the treatment of multi-drug resistant strains of *falciparum* malaria, either dihydroartemisinin or a prodrug thereof such as artemisinin, artesunate, artemether, artelinic acid, artenimol or artemotil is used. The compounds can be isolated from the plant *Artemisia annua* and/or chemically synthesized.

There is a continuous need in the art for novel pharmaceutical compositions for the treatment of cancer, and especially hematological malignancies such as multiple myeloma. Accordingly, it is a goal of the present invention, amongst other goals, to provide novel pharmaceutical compositions for the treatment of cancer, and especially hematological malignancies such as multiple myeloma.

This goal, amongst other goals, is met by the present invention by a pharmaceutical composition as defined in the appended claim 1.

Especially, this goal, amongst other goals, is met by a pharmaceutical composition for the treatment of cancer comprising:
- thalidomide, or a derivative thereof, or salts or solvates thereof ;
- one or more artemisinin compounds, or salts or solvates thereof; and
- one or more pharmaceutically acceptable carriers and/or excipients.

It was surprisingly discovered by the present inventor that pharmaceutical compositions as defined above provided a synergistic anti-cancer activity which could not be attributable to the separate activities of the constituents of the composition, i.e., thalidomide and dihydroartemisinin.

Thalidomide, or a derivative thereof, comprises racemic thalidomide, stereomerically enriched or stereomerically pure thalidomide, or a derivative thereof, and pharmaceutically acceptable salts, solvates, hydrates, stereoisomers, clathrates, and prodrugs thereof.

Suitable organic acids to form the present pharmaceutically acceptable salts comprise, for example, maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (i. e., 1, l'-methylene-bis- (2-hydroxy-3-naphthoate) acids. Suitable inorganic acids comprise, for example, hydrochloric, hydrobromic, hydroiodic, sulfuric, phosphoric, or nitric acids.

Suitable organic bases to form the present pharmaceutically acceptable salts comprise, for example, N, N-dibenzylethylenediamine, chloroprocaine, choline, diethanolamine, ethylenediamine, meglumaine (N-methylglucamine), lysine, and procaine.

A pharmaceutical composition according to the present invention can be used for the preparation of individual, single unit dosage forms.

Pharmaceutical compositions and dosage forms according to the present invention are suitable for oral, mucosal, sublingual, vaginal, buccal, or rectal, parenteral, subcutaneous, intravenous, bolus injection, intramuscular, or intra-arterial, transdermal or transcutaneous administration.

Examples of dosage forms comprise, for example, tablets, caplets, capsules, such as soft elastic gelatin capsules, cachets, troches, lozenges, dispersions, suppositories, powders, aerosols, gels, liquid dosage forms suitable for oral or mucosal administration such as aqueous or non-aqueous liquid suspensions, oil-in-water emulsions, or a water-in-oil liquid emulsions, solutions, or elixirs.

Suitable excipients according to the present invention comprise, for example, water, glycols, oils, alcohols, flavoring agents, preservatives, coloring agents, starches, sugars, micro-crystalline cellulose, diluents, granulating agents, lubricants, fillers, binders, and/or disintegrating agents.

Examples of binders comprise corn starch, potato starch, or other starches, gelatin, natural and synthetic gums such as acacia, sodium alginate, alginic acid, other alginates, powdered tragacanth, guar gum, cellulose, ethyl cellulose, cellulose acetate, carboxymethyl cellulose calcium, sodium carboxymethyl cellulose, polyvinyl pyrrolidone, methyl cellulose, pre-gelatinized starch, hydroxypropyl methyl cellulose, microcrystalline cellulose, and mixtures thereof.

Examples of fillers comprise talc, calcium carbonate, microcrystalline cellulose, powdered cellulose, dextrates, kaolin, mannitol, silicic acid, sorbitol, starch, pre-gelatinized starch, and mixtures thereof.

Examples of disintegrating agents comprise agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Examples of lubricants comprise calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil, peanut oil, cottonseed oil, sunflower oil, sesame oil, olive oil, corn oil, and soybean oil, zinc stearate, ethyl oleate, ethyl laureate, agar, and mixtures thereof.

According to a preferred embodiment, the present invention relates to pharmaceutical compositions wherein the present derivative of thalidomide is chosen from the group consisting of lenalidomide, bortezomib, actimid, CC-5013 and CC-4047, although other derivates having an anti-cancer activity, such as an anti-angiogenesis or anti-proliferative activity, are also encompassed by the present invention.

According to another preferred embodiment, the present invention relates to a pharmaceutical composition, wherein the present one ore more artemisinin compound are chosen from the group consisting of dihydroartemisinin, artemisinin, artesunate, artemether, artelinic acid, artenimol and artemotil, although other artemisinin compounds having a sesquiterpene lactone structure are encompassed by the present invention.

According to yet another preferred embodiment, the present invention relates to a pharmaceutical composition, wherein the present one or more artemisinin compound is an artemisinin derivative according to the general formula (I) 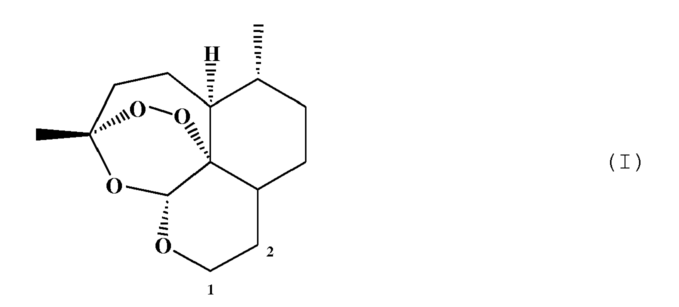 substituted at the 1 and/or 2 position with a moiety selected from the group consisting of methyl N-(1H-benzimidazol-2-yl)carbamate, methoxyacetetic acid, 2,2-dichloroacetate, 5-fluoro-1H-pyrimidine-2,4-dione, acetate, butyrate, n-butyrate, iso-butyrate, propionate, n-propionate, propylpentanoate, valproate, isopropyl ester, polyalcohol-acids, glyceric acid (2,3-dihydroxypropionic acid, 2-hydroxymethyl-3-hydroxypropionic acid, 3,4-dihydroxybutyric acid, 2-hydroxymethyl-3,4-dihydroxybutyric acid, 1,3-dihydroxypropane-1,2-dicarboxylic acid, polyalcohol-acids, sugar-acids, and glucuronic acid.

According to still another preferred embodiment, the present invention relates to a pharmaceutical composition, wherein the present artemisinin compound is an artemisinin derivative according to the general formula (I) 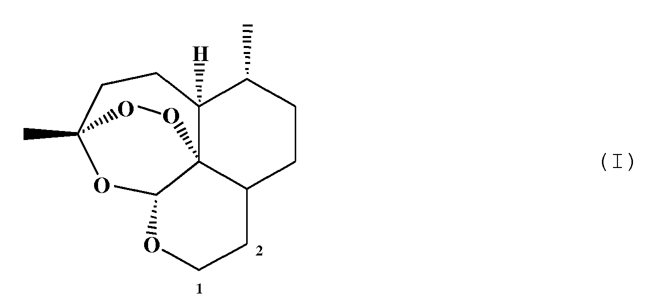 substituted at the 1 and/or 2 position with a C₁ to C₈ alkyl or aryl moiety, branched or unbranched, substituted or non-substituted.

The present moieties are preferably covalently linked to the artemisinin compound by a sulfide (-S-), ether (-O-), ester (-OCO-) or amine (-N-) linkage.

According to an especially preferred embodiment of the present invention, the present moiety is covalently linked to the present artemisinin compound at the 1 position.

According to another especially preferred embodiment of the present invention, the present moiety is covalently linked to the present artemisinin compound at the 2 position.

According to the present invention, the compound according to formula (I) is preferably derived from a compound selected from the group consisting of dihydroartemisinin, anhydrodihydroartemisinin and deoxartemisinin.

The compound according to formula (I) can be derived from a known intermediate of the biosynthesis of the antimalarial drug artemisinin, i.e., dihydroartemisinin (DHA).

In case of dihydroartemisinin, the 1 position of the initial artemisinin derivative comprises an -OH group allowing covalent coupling of a present moiety to the 1 position, for example, by an estrification reaction or an etherification reaction. Such reactions are common general knowledge in the field of organic chemistry and can be found in most organic chemistry text books such as, for example, Organic Chemistry, John McMurry, Brooks/Cole, 6^{th} edition.

Another starting compound for providing the compound according to formula (I) is deoxartemisinin. In this starting compound, a possible coupling group, such as -OH, is provided at the 2 position.

Another starting compound for the compound according to formula (I) is anhydrodihydroartemisinin (AHA). This compound comprises a double bond between the 1 and 2 position allowing, through an intermediate reaction, covalently coupling of a biologically active compound to the 1 position or the 2 position, for example, by a nucleophile reaction of O, N or S to provide the corresponding derivatives at the 1 position, or a hydroxylation reaction by an anti-markofnikoff addition at position 2.

Such reactions are common general knowledge in the field of organic chemistry and can be found in most organic chemistry text books such as, for example, Organic Chemistry, John McMurry, Brooks/Cole, 6th edition.

Accordingly, according to a preferred embodiment of the present invention, an artemisinin compound according to the present invention is derived from a compound chosen from the group consisting of dihydroartemisinin, anhydrodihydroartemisinin and deoxartemisinin

According to a more preferred embodiment of the present invention, a pharmaceutical composition comprises an artemisinin compound according to the formula (II):

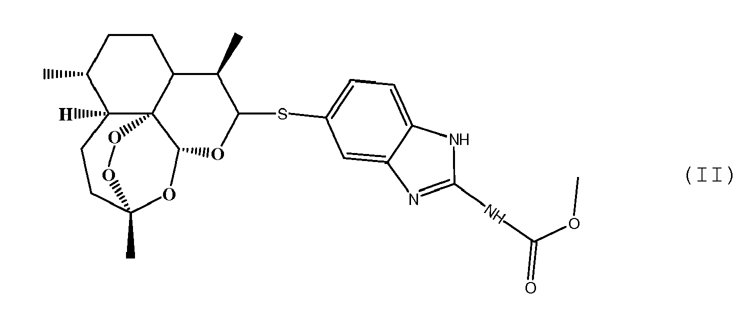

According to another more preferred embodiment of the present invention, a pharmaceutical composition comprises an artemisinin compound according to the formula (III):

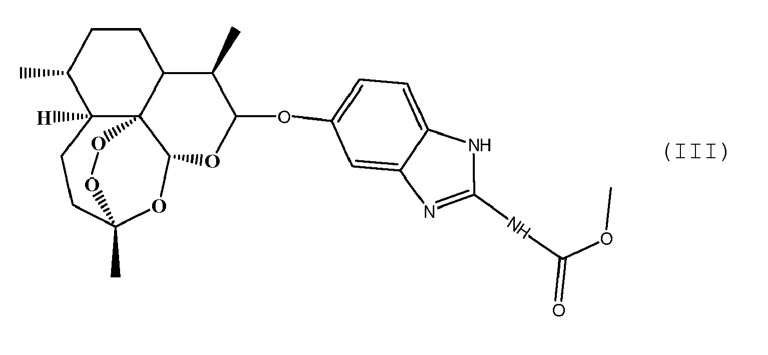

According to yet another more preferred embodiment of the present invention, a pharmaceutical composition comprises an artemisinin compound according to the formula (IV) :

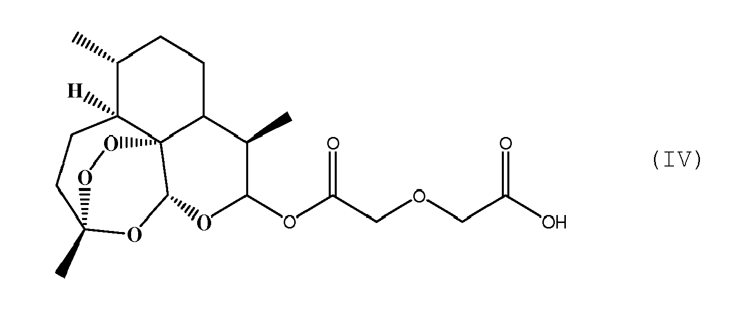

According to still another more preferred embodiment of the present invention, a pharmaceutical composition comprises an artemisinin compound according to the formula (V) :

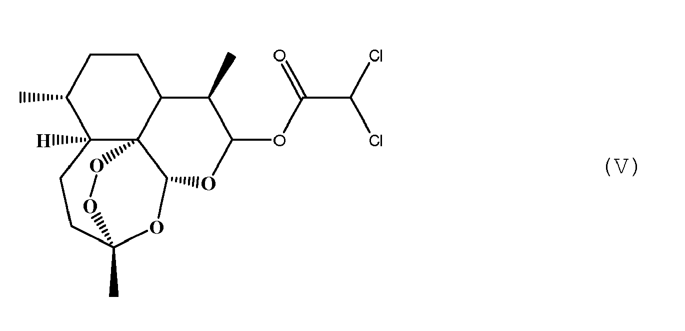

According to a further more preferred embodiment of the present invention, a pharmaceutical composition comprises an artemisinin compound according to the formula (VI) :

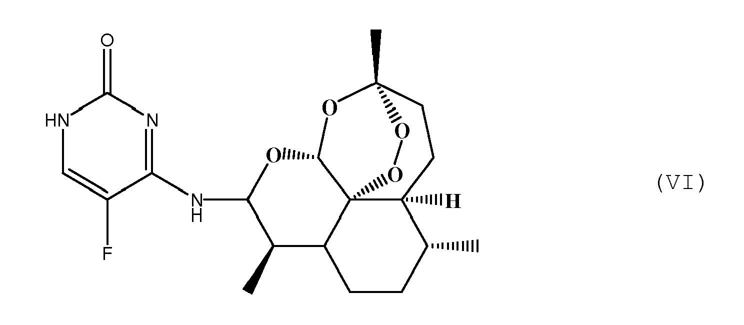

According to a yet a further more preferred embodiment of the present invention, a pharmaceutical composition comprises an artemisinin compound according to the formula (VII):

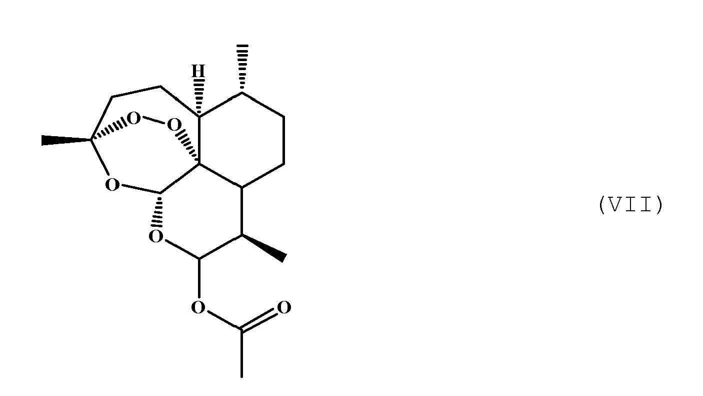

According to a still a further more preferred embodiment of the present invention, a pharmaceutical composition comprises an artemisinin compound according to the formula (VIII):

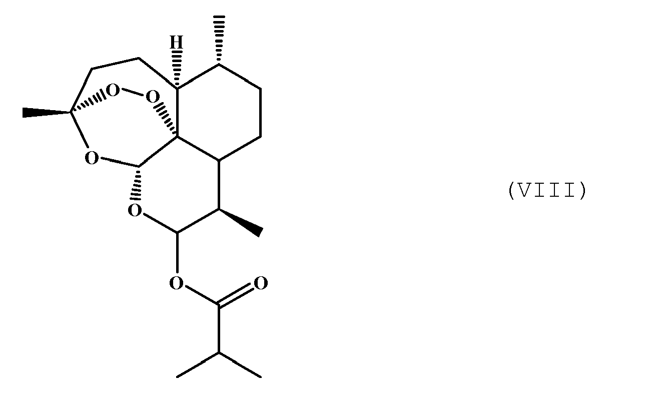

According to a yet another further more preferred embodiment of the present invention, a pharmaceutical composition comprises an artemisinin compound according to the formula (IX):

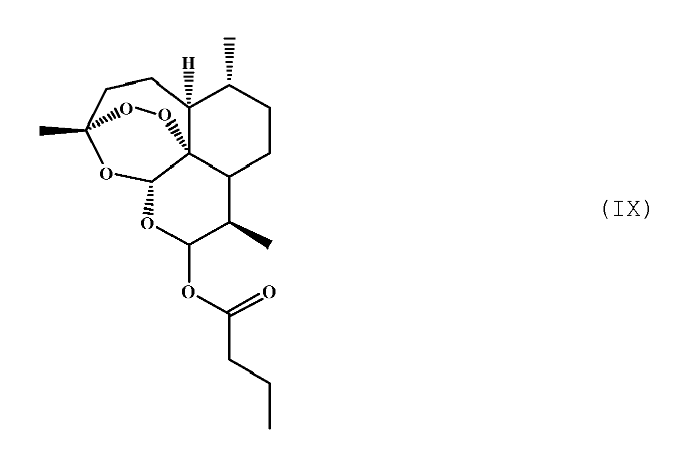

According to a still another further more preferred embodiment of the present invention, a pharmaceutical composition comprises an artemisinin compound according to the formula (X):

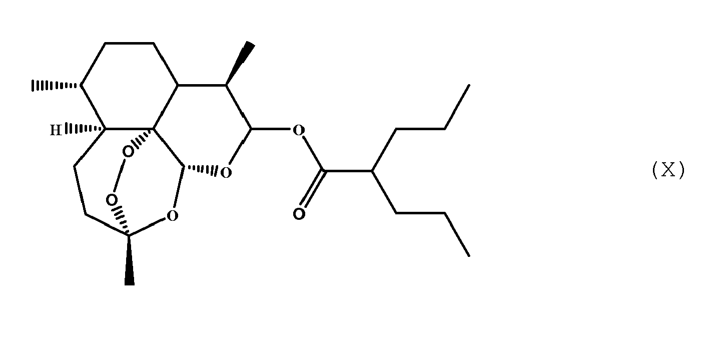

According to a subsequent further more preferred embodiment of the present invention, a pharmaceutical composition comprises an artemisinin compound according to the formula (XI): 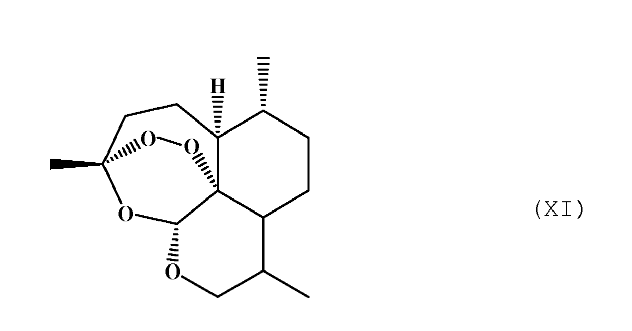 or an artemisinin compound according to the formula (XII): 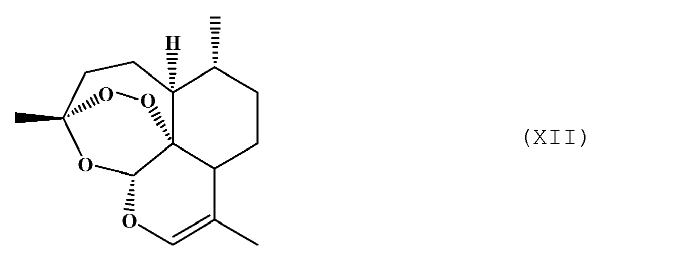 or an artemisinin compound according to the formula (XIII): 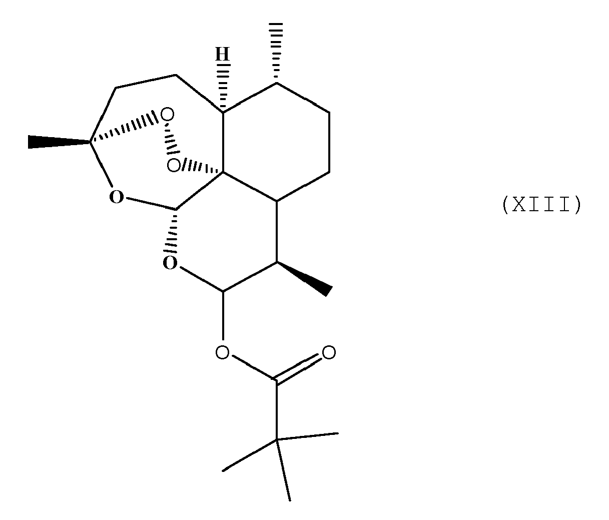 or an artemisinin compound according to the formula (XIV): 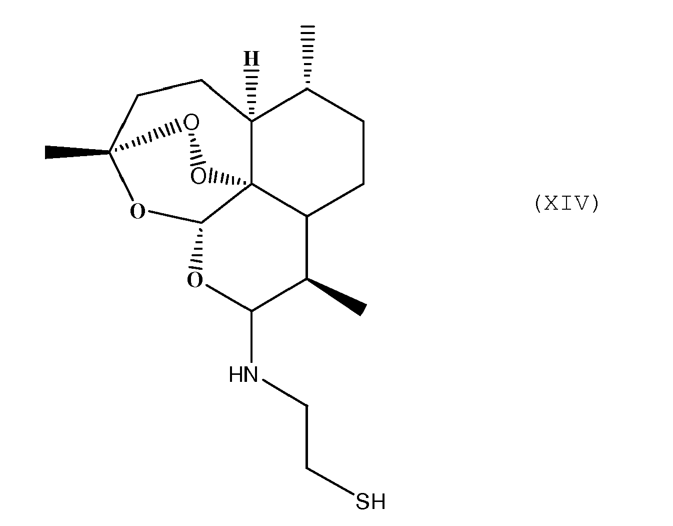 or an artemisinin compound according to the formula (XV): 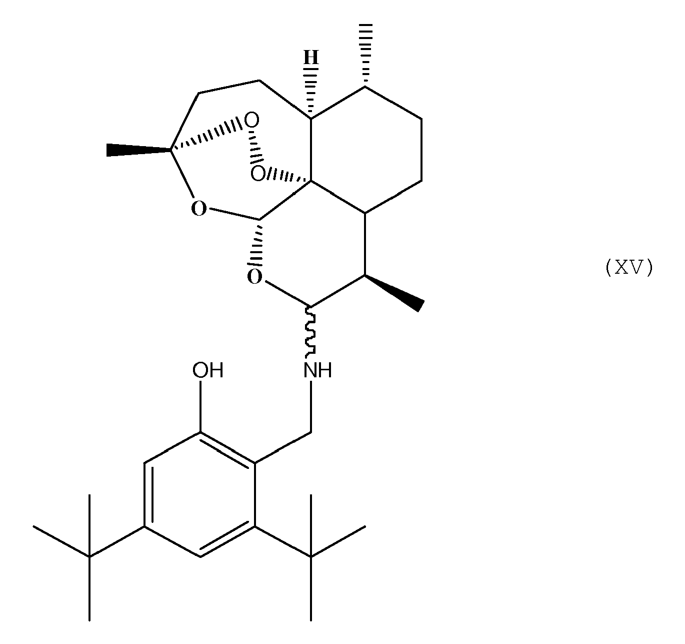 or an artemisinin compound according to the formula (XVI):

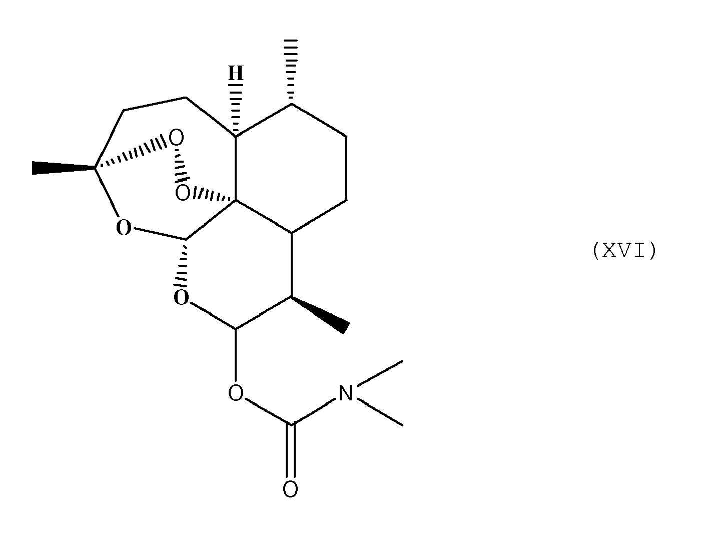

The present pharmaceutical composition is preferably used for the treatment of a cancer chosen from the group consisting of carcinoma, sarcoma, germ cell, blastoma and hematological, preferably a hematological malignancy.

The present pharmaceutical composition is more preferably used for the treatment of a hematological malignancy chosen from the group consisting of lymphomas, acute lymphoblastic leukemia, myeloma, acute and chronic myelogenous leukemia, myelodysplastic syndromes and myeloproliferative diseases, most preferably multiple myeloma.

The present invention, according to another aspect, relates to the use of the present pharmaceutical compositions for the treatment of the present cancers.

The present invention, according to yet another aspect, relates to the use of thalidomide, or a derivative thereof and one or more of the present artemisinin compounds for the treatment of the present cancers.

The present invention will be further detailed in the following examples of preferred embodiments of the present invention.

### Examples

### Example 1: General synthesis of an artemisinin compound according to the present invention

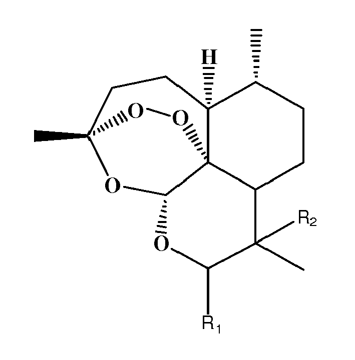

wherein R₁ and R₂ are H or a biologically active molecule according to the present invention under the provision that at least R₁ or R₂ is a biologically active molecule according to the present invention.

In a three neck flask containing 75 ml of liq. NH₃ was added approximately 0.1 g of Na in small pieces, then a methylbenzimidazole derivative 2.37 g (10 mmol) was slowly added and the mixture was set for stirring. The reaction was monitored by thin layer chromatography by taking small amounts out of reaction mixture.

After 2 hours, the reaction was complete and subsequently quenched through the addition of ammonium chloride and the ammonia was allowed to evaporate.

Excess of Na was neutralized by the careful addition of water and the resulting product was repeatedly extracted with ether and recrystallized from dichloromethane/hexane yielding 1.8 g (8.07 mmol, 80.7%) of the desired product.

### Example 2: Synthesis of the thiobenzimidazole derivative: Methyl 5-(dihydroartemisinylthio)-1H-benzo[d]imidazol-2-ylcarbamate (1a)

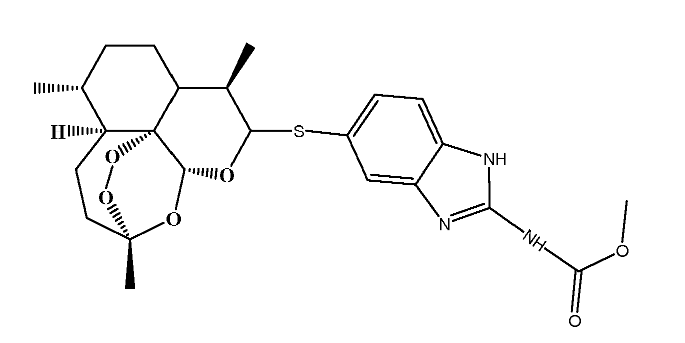

Boron trifluoride-diethyl ether (1 ml) was added to a stirred solution of dihydroartemisinin (DHA, 2.56 g, 9 mmol) and benzimidazole derivative (methyl N-(1H-benzimidazol-2-yl)carbamate, 1.80 g, 8.07 mmol) in dry diethyl ether (50 ml). The mixture was stirred at room temperature overnight and subsequently quenched with saturated aqueous NaHCO₃ and dried over MgSO₄.

Filtration and concentration of the filtrate provided a residue which on chromatography with ethyl acetate/hexane (10:90) provided the product as yellow solid (mixture of epimers). The yield was 2.37 g (70%), m.p. 168-170°C.
1H NMR: 0.85-1.81 (m, 17 H), 1.97-2.03 (m, 1 H), 2.13-2.23 (m, 1H), 2.23-2.28 (m, 0.5H),3.75 (s, 3H), 4.78 (s), 4.81 (s), 5.41 (s, 1H), 5.56-5.66 (two singlet's, 1H), 7.18-7.38 (m, 2H), 7.55-7.62 (m, 1H)

### Example 3: Synthesis of methyl 5-(dihydroartemisinyloxy)-1H-benzo[d]imidazol-2-ylcarbamate (1b)

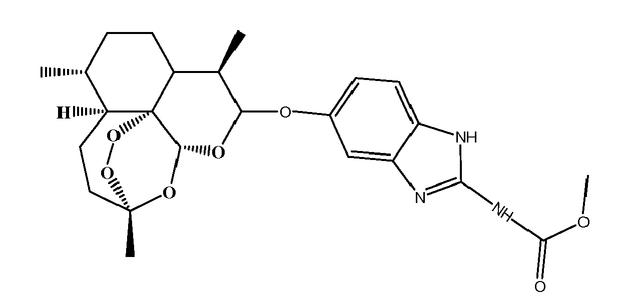

3.42 g (12 mmol) DHA was reacted with methyl N-(1H-benzimidazol-2-yl)carbamate (1.94 g, 10 mmol) using the method describe in example 2, except that the eluent for chromatography was ethyl acetate/hexane gradient mode (5:95-10:90).

The product obtained was further recrystallized from dichloromethane (mixture of epimers) as a yellow solid (2.98 g, 63%), m.p. 172-175°C
1H NMR: 0.86-1.80 (m, 17 H), 1.99-2.02 (m, 1 H), 2.13-2.23 (m, 1H), 2.23-2.28 (m, 0.5H), 3.71 (s, 3H), 4.78 (s), 5.50 (m, 1H), (s), 5.41 (s, 1H), 5.56-5.58 (two singlets, 1H), 6.78-6.90 (m, 1H), 7.25-7.69 (m, 2H).

### Example 4: Synthesis of 2-((dihydroartemisinylcarbonyl)methoxy)acetic acid (2a)

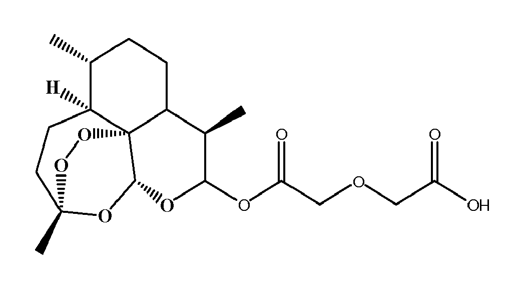

4-(Dimethylamino)pyridine (1.95 g, 16 mmol) and diglycolic anhydride (3.48 g, 30 mmol) were added to a stirred solution of DHA (2.84 g, 10 mmol) in dichloromethane (50 ml) and the reaction mixture was continuously stirred overnight.

The solvent was removed under reduced pressure and the residue was purified by column chromatography with ethyl acetate/hexane (10:90) to provide the product as a white solid (3.80 g, 95%). Recrystallization from ethyl acetate/hexane provided white needles (m.p. 168-171°C)
1HNMR 0.86 6 (d, J = 7.0 Hz, 3 H, 9-Me), 0.97 (d, J = 5.95 Hz, 3 H, 6-Me), 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 3.0 Hz, 1 H), 2.55 (m, 1H, H-9),4.25 (s, 2 H, OCH2COO), 5.40 (s, 2H, COCH2O)5.45 (s, 1H, H-12), 5.850 (d, J = 10.0 Hz, 1 H, H-10)

### Example 5 Synthesis of dihydroartemisinyl-2,2-dichloroacetate

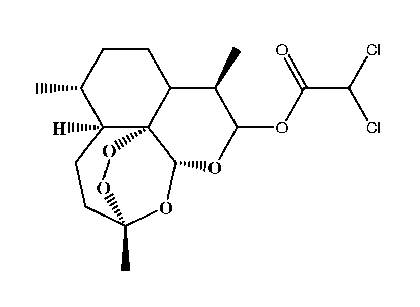

4-(Dimethylamino)pyridine (1.95 g, 16 mmol), diglycolic anhydride (3.48 g, 30 mmol) and DHA (2.84 g, 10 mmol) were used for the synthesis using the above method except that the eluent for chromatography was ethyl acetate/hexane (5:95). The product was obtained as yellow solid (3.44 g, 87%), m.p. 160-163°C
1HNMR 0.86 (d, J = 7.0 Hz, 3 H, 9-Me), 0.97 (d, J = 5.95 Hz, 3 H, 6-Me), 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 3.0 Hz, 1 H), 2.55 (m, 1H, H-9), 5.40 (s, 1H, H-12), 5.90 (d, J = 10.0 Hz, 1 H, H-10), 6.25 (s, 1 H, COCHCl2).

### Example 6: Synthesis of 5-Fluoro-4-(dihydroartemisinly amino)pyrimidin-2(1H)-one

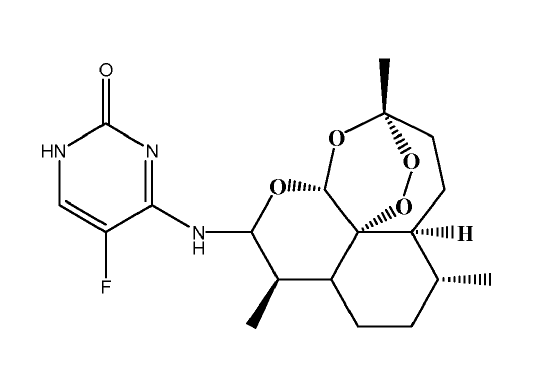

Boron trifluoride-diethyl ether (1 ml) was added to a stirred solution of DHA (2.56 g, 9 mmol) and F-uracil (1.04 g, 8.05 mmol) in dry diethyl ether (50 ml). The mixture was stirred at room temperature overnight and subsequently quenched with saturated aqueous NaHCO₃ and dried over MgSO₄.

Filtration and concentration of the filtrate provided a residue which on chromatography with ethyl acetate /hexane (10:90) provided the product as a yellow solid (mixture of epimers, 2.36 g, 74%), m.p. 165-17°C.
1HNMR 0.86 (d, J = 7.0 Hz, 3 H, 9-Me), 0.97 (d, J = 5.95 Hz, 3 H, 6-Me), 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 3.0 Hz, 1 H), 2.55 (m, 1H, H-9), 5.40 (s, 1H, H-12), 5.90 (d, J = 10.0 Hz, 1 H, H-10),

### Example 7: Synthesis of anhydrodihydroartemisinin (AHA)

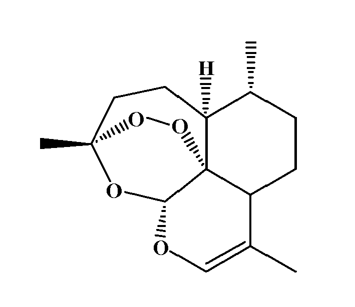

Boron trifluoride-diethyl ether (3 ml) was added to a stirred solution of DHA (2.56 g, 9 mmol) at 0°C and the mixture was slowly brought to room temperature, stirred for 2 hours and subsequently quenched with saturated aqueous NaHCO₃ and dried over MgSO₄.

Filtration and concentration of the filtrate provided a residue which on chromatography with ethyl acetate /hexane (10:90) provided the product as white solid (2.15 g, 90%), m.p. 95-98°C
1HNMR 0.98 (d, J = 5.8 Hz, 3 H, 6-Me), 1.02-1.39 (m, 2 H), 1.42 (m, 3 H, 9-Me), 1.44-1.75 (m, 8 H), 1.87-1.96 (m, 1 H), 2.00-2.12 (m, 2 H), 2.35-2.46 (m, 1 H), 5.54 (s, 1 H, H-12), 6.18 (s, J _ 1 H, 10-H) ppm.

### Example 8: Synthesis of deoxartemisinin

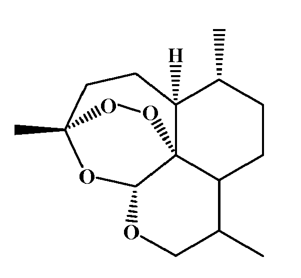

A solution of dihydroartemisinin (ADH, 456 mg, 1.6 mmol) in dry CH₂Cl₂ (16 ml) in a 50 ml round bottom flask under an argon atmosphere was cooled to -2°C. To this solution were added triethylsilane (0.40 ml, 2.4 mmol) and boron trifluoride etherate (0.24 ml, 1.92 mmol). The solution was then allowed to warm to 5°C over 2 h., and subsequently 15 ml of water was added.

The organic layer was separated, washed several times with water and dried over Na₂SO₄. The solution was concentrated and the crude product was purified by flash chromatography (hexane/CH₂Cl₂/6:4) to provide 380 mg pure product (88%), m.p. 104-106°C

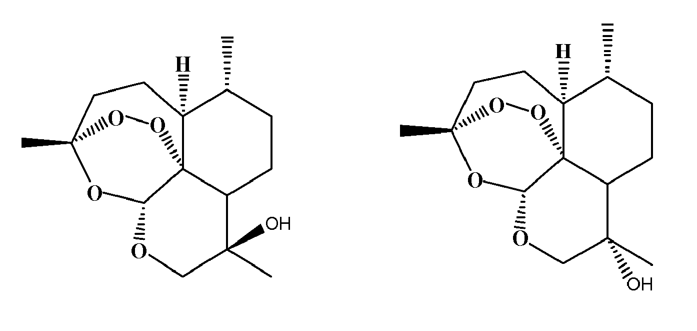

To a solution of anhydrodihydroartemisinin (AHA) (2.08 g, 7.8 mmol) in THF (20 ml), was drop wise added a solution of BH₃/THF complex in THF solution (1 M; 14 ml) with ice-cooling. After being stirred at room temperature for 1 h., the mixture was treated with THF-water (1:1; 4 ml), followed by a mixture of aq. KOH (10%; 10 ml) and aqueous H₂O₂ (50%; 4 ml) .

Thereafter, the reaction mixture was stirred for 5 min, filtered and evaporated. The residue was washed with water and taken up into Et₂O and the ethereal solution was dried (MgSO₄) and evaporated to provide a white solid of a mixture of isomers which were separated by column chromatography (hexane/ethyl acetate).

### Example 9: Synthesis of dihydroartemisinylacetate

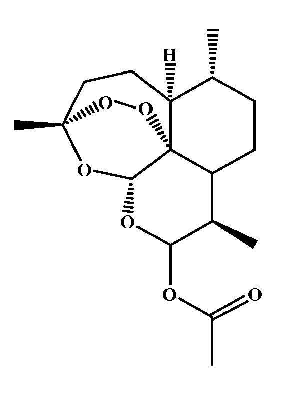

4-(Dimethylamino)pyridine (0.5 g, 4.1 mmol) and acetic anhydride anhydride (3.06 g, 30 mmol) were added to a stirred solution of DHA (7.1 g, 25 mmol) in dichloromethane (400 ml) and the reaction mixture was continuously stirred overnight. The solvent was removed under reduced pressure and the residue was purified by column chromatography with ethyl acetate/hexane (10:90) to provide the product as a white solid (6.80 g, 84%). Recrystallization from ethyl acetate/hexane provided white needles (m.p. 129-131°C).
1HNMR: 0.86 (d, J = 7.0 Hz, 3 H, 9-Me), 0.97 (d, J = 5.95 Hz, 3 H, 6-Me), 1.45 (s, 3 H, 3-Me), 1.23-1.94 (m, 9 H), 2.04 (ddd, J = 14.5, 5.0, 3.0 Hz, 1H), 2.12 (s, 3 H, H-COCH3), 2.39 (ddd, J = 14.5, 5.0, 15 3.0 Hz, 1 H), 2.55 (m, 1H, H-9), 5.45 (s, 1H, H-12), 5.850 (d, J = 10.0 Hz, 1 H, H-10).

### Example 10: Synthesis of dihydroartemisinyl iso-butyrate

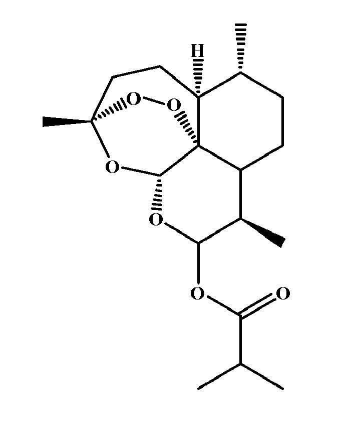

4-(Dimethylamino)pyridine (0.6 g, 4.9 mmol) and isobutyric anhydride (4.0 g, 25 mmol) were added to a stirred solution of DHA (5 g, 17.6 mmol) in dichloromethane (200 ml) and the reaction mixture was continuously stirred overnight. The solvent was removed under reduced pressure and the residue was purified by column chromatography with ethyl acetate/hexane (10:90) to provide the product as a dense liquid (6.80 g, 84%).
1HNMR: 0.86 (d, J = 7.0 Hz, 3 H, 9-Me), 0.97 (d, J = 5.95 Hz, 3 H, 6-Me), 1.17-1.24 (m, 6 H) 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 15 3.0 Hz, 1 H), 2.55 (m, 1H, H-9), 2.68 (m, 1H, COCH), 5.45 (s, 1H, H-12), 5.850 (d, J = 10.0 Hz, 1H, H-10).

### Example 11: Synthesis of dihydroartemisinylbutyrate

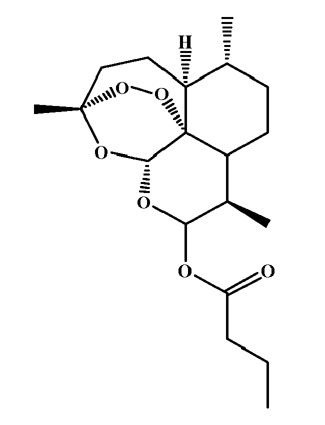

4-(Dimethylamino)pyridine (0.6 g, 4.9 mmol) and Isobutyric anhydride (4.0 g, 25 mmol) were added to a stirred solution of DHA (5 g, 17.6 mmol) in dichloromethane (300 ml) and the reaction mixture was continuously stirred overnight. The solvent was removed under reduced pressure and the residue was purified by column chromatography with ethyl acetate/hexane (10:90) to provide the product as a dense liquid (5.9 g, 95%).
1HNMR: 0.86 (d, J = 7.0 Hz, 3 H, 9-Me), 0.97 (d, J = 5.95 Hz, 3 H, 6-Me), 1.17-1.24 (m, 6 H), 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 15 3.0 Hz, 1 H), 2.55 (m, 1H, H-9), 2.68 (m, 1H, COCH), 5.45 (s, 1H, H-12), 5.850 (d, J = 10.0 Hz, 1H, H-10).

### Example 12: Synthesis of dihydroartemisinyl-2-propylpentanoate

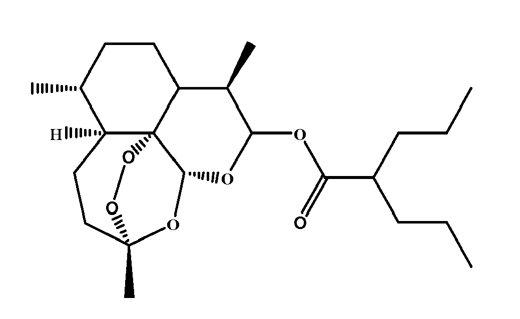

4-(Dimethylamino)pyridine (0.5 g, 4.1 mmol) and triethylamine (3.03g, 30 mmol) were added to a stirred solution of DHA (7.1 g, 25 mmol) in dichloromethane (400 ml). To this solution was added 2-Proplypentanlychlorid (4.87 g, 30 mmol) at -30°C, the reaction mixture was continuously stirred for 2 hours, slowly brought to room temperature and stirred overnight.

The solvent was removed under reduced pressure and the residue was purified by column chromatography with ethyl acetate/hexane (10:90) to provide the product as a white solid (10.22 g, 80%). Recrystallization from ethyl acetate/hexane provided white needles (m.p. 141-145°C).
1HNMR: 0.86 (d, J = 7.0 Hz, 3 H, 9-Me), 0.90 (t, 6H), 0.97 (d, J = 5.95 Hz, 3 H, 6-Me), 1.33 (m, 4H), 1.45 (s, 3 H, 3-Me), 1.64 (m, 4H), 1.23-1.94 (m, 9 H), 2.04 (ddd, J = 14.5, 5.0, 3.0 Hz, 1H), 2.29 (t, 1H), 2.39 (ddd, J = 14.5, 5.0, 15 3.0 Hz, 1 H), 2.55 (m, 1H, H-9), 5.45 (s, 1H, H-12), 5.850 (d, J = 10.0 Hz, 1 H, H-10).

### Example 13: Synthesis of dihydroartemisinyl 2,2 dimethylpropianate

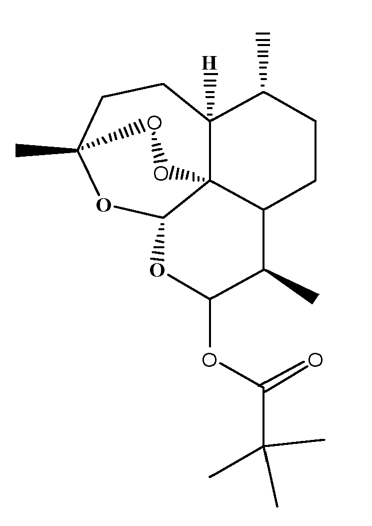

4-(Dimethylamino)pyridine (0.5 g, 4.1 mmol) and trimethylacetic anhydride (5.59 g, 30 mmol) were added to a stirred solution of DHA (7.1 g, 25 mmol) in dichloromethane (400 ml) and the reaction mixture was continuously stirred overnight. The crude mixture was washed with water (2 x 100 ml) and solvent was removed under reduced pressure and the product was recrystallized from ethyl acetate/hexane providing a white solid which melts at 101-104°C (6.9 g, 750) .
1HNMR: 0.86 (d, J = 7.0 Hz, 3 H, 9-Me), 0.97 (d, J = 5.95 Hz, 3 H, 6-Me), 1.25 (s, 9H C(CH)3), 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 15 3.0 Hz, 1 H), 2.55 (m, 1H, H-9), 5.45 (s, 1H, H-12), 5.850 (d, J = 10.0 Hz, 1 H, H-10).

### Example 14: Synthesis of dihydroartemisinylthioethyl

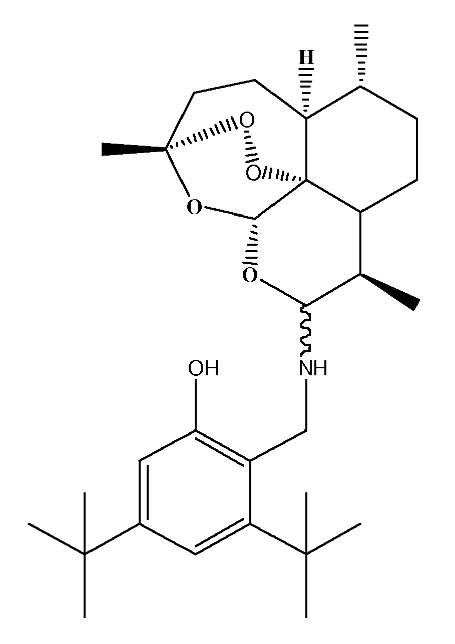

DHA (7.1 g, 25 mmol) and cystamine (2.7 g, 35 mmol) were dissolved in 300 ml dichloromethane and borontrifluoride-diethyl ether (10 ml) was slowly added at 0EC. The reaction mixture stirred for 3 hours at 0°C and subsequently 1 hour at room temperature. The reaction was quenched with 5% NaHCO₃ and extracted with dichloromethane. The solvent was removed under reduced pressure and the residue was purified by column chromatography with ethyl acetate/hexane (10:90) to provide the product as a brown wax, yield 6.5 g.
1HNMR: 0.86 (d, J = 7.0 Hz, 3 H, 9-Me), 0.97 (d, J = 5.95 Hz, 3 H, 6-Me), 1.25, 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 15 3.0 Hz, 1 H), 2.55 (m, 1H, H-9), 2.9 (t, 2H), 3.1 (t, 2H), 4.56 (d, J = 10.0 Hz, 1 H, H-10), 5.31 (s, 1H, H-12).

### Example 15: Synthesis of 3,5-di-tert-butyl-2-(dihydroartemisinylaminomethyl)phenol

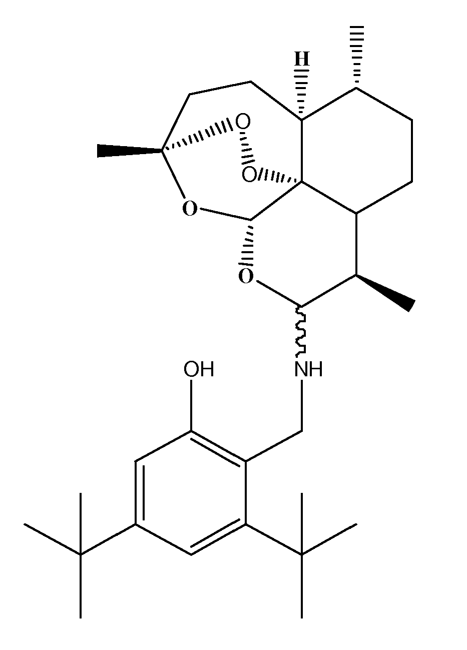

DHA (7.1 g, 25 mmol) and 3,5-di-tert-butyl-2-(aminomethyl)phenol (7.06 g, 30 mmol) were dissolved in 300 ml dichloromethane and borontrifluoride-diethyl ether (10 ml) was added slowly at 0°C. The reaction mixture was stirred for 3 hours at 0°C and subsequently 1 hour at room temperature. The reaction was quenched with 5% NaHCO₃ and extracted with dichloromethane. The solvent was removed under reduced pressure and the residue was purified by column chromatography with ethyl acetate/hexane (20:80) to provide the product as a solid, yield 6.0 g.
1HNMR: 0.86 (d, J = 7.0 Hz, 3 H, 9-Me), 0.97 (d, J = 5.95 Hz, 3 H, 6-Me),1.34 4 (s, 18H), 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 15 3.0 Hz, 1 H), 2.55 (m, 1H, H-9), 3.91 (s, 2H), 5.850 (d, J = 10.0 Hz, 1 H, H-10), 5.45 (s, 1H, H-12), 6.44 (d, 1H), 6.76 (d, 1H).

### Example 16: Synthesis of dihydroartemisinyl N,N-dimethylacetamide

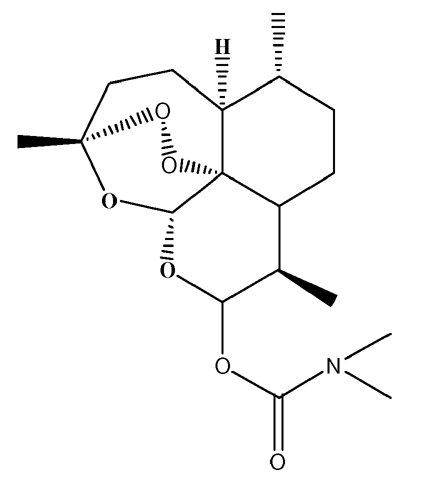

4-(Dimethylamino) pyridine (0.5 g, 4.1 mmol) and dimethylcarbomoyl chloride (3.23 g, 30 mmol) were added to a stirred solution of DHA (7.1 g, 25 mmol) in dichloromethane (400 ml) and the reaction mixture was continuously stirred overnight. The crude was washed with water (2 x 100 ml) and solvent was removed under reduced pressure and the product was recrystallized from ethyl acetate/hexane provided yellow solid, yield 8.0 g (90 %).
1HNMR: 0.86 (d, J = 7.0 Hz, 3 H, 9-Me), 0.97 (d, J = 5.95 Hz, 3 H, 6-Me), 1.45 (s, 3 H, 3-Me), 1.23-1.94 (m, 9 H), 2.04 (ddd, J = 14.5, 5.0, 3.0 Hz, 1H), 2.39 (ddd, J = 14.5, 5.0, 15 3.0 Hz, 1 H), 2.55 (m, 1H, H-9), 2.90 (s, 6H, N(CH3)2 5.45 (s, 1H, H-12), 5.850 (d, J = 10.0 Hz, 1 H, H-10).

## Claims

1. Pharmaceutical composition for the treatment of cancer comprising:
- thalidomide, or a derivative thereof, or salts or solvates thereof ;
- one or more artemisinin compounds, or salts or solvates thereof; and
- one or more pharmaceutically acceptable carriers and/or excipients.

2. Pharmaceutical composition according to claim 1, wherein said derivative of thalidomide is chosen from the group consisting of lenalidomide, bortezomib, actimid, CC-5013 and CC-4047.

3. Pharmaceutical composition according to claim 1 or claim 2, wherein said one ore more artemisinin compounds are chosen from the group consisting of dihydroartemisinin, artemisinin, artesunate, artemether, artelinic acid, artenimol and artemotil.

4. Pharmaceutical composition according to claim 1 or claim 2, wherein said one or more artemisinin compounds are an artemisinin derivative according to the general formula (I) 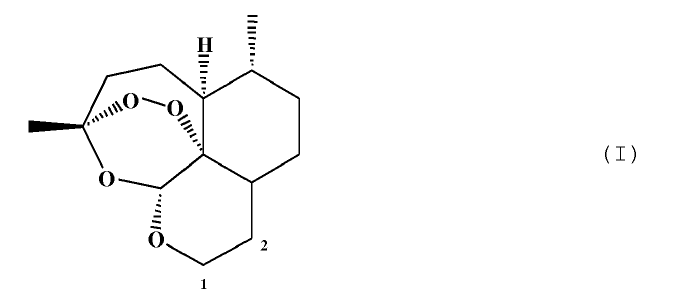 substituted at the 1 and/or 2 position with a moiety selected from the group consisting of methyl N-(1H-benzimidazol-2-yl)carbamate, methoxyacetetic acid, 2,2-dichloroacetate, 5-fluoro-1H-pyrimidine-2,4-dione, acetate, butyrate, n-butyrate, iso-butyrate, propionate, n-propionate, propylpentanoate, valproate, isopropyl ester, polyalcohol-acids, glyceric acid (2,3-dihydroxypropionic acid, 2-hydroxymethyl-3-hydroxypropionic acid, 3,4-dihydroxybutyric acid, 2-hydroxymethyl-3,4-dihydroxybutyric acid, 1,3-dihydroxypropane-1,2-dicarboxylic acid, polyalcohol-acids, sugar-acids, and glucuronic acid.

5. Pharmaceutical composition according to claim 1 or claim 2, wherein said one or more artemisinin compounds are an artemisinin derivative according to the general formula (I) 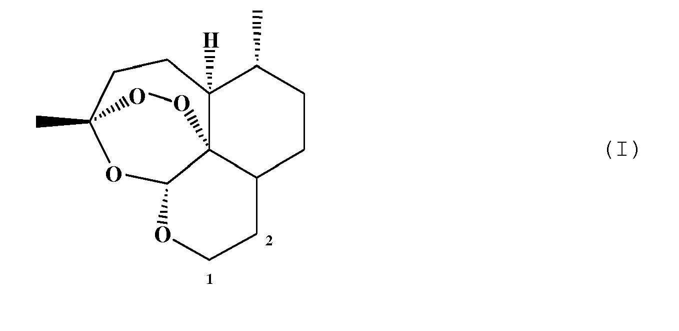 substituted at the 1 and/or 2 position with a C₁ to C₈ alkyl or aryl moiety, branched or unbranched, substituted or not substituted.

6. Pharmaceutical composition according to claim 4 or claim 5, wherein the moiety is covalently linked to the artemisinin compound through a sulfide (-S-), ether (-O-), ester (-OCO-) or amine (-N-) linkage.

7. Pharmaceutical composition according to any of the claims 4 to 6, wherein the moiety is covalently linked to the artemisinin compound at the 1 position.

8. Pharmaceutical composition according to any of the claims 4 to 6, wherein the moiety is covalently linked to the artemisinin compound at the 2 position.

9. Pharmaceutical composition according to any of the claims 4 to 8, wherein the artemisinin derivative is derived from a compound selected from the group consisting of dihydroartemisinin, anhydrodihydroartemisinin and deoxartemisinin.

10. Pharmaceutical composition according to claim 1 or claim 2, wherein one or more artemisinin compounds are a compound according the formula (II):

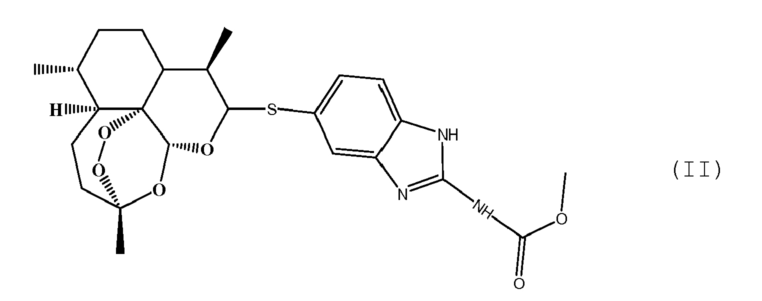

11. Pharmaceutical composition according to claim 1 or claim 2, wherein one or more artemisinin compounds are a compound according the formula (III):

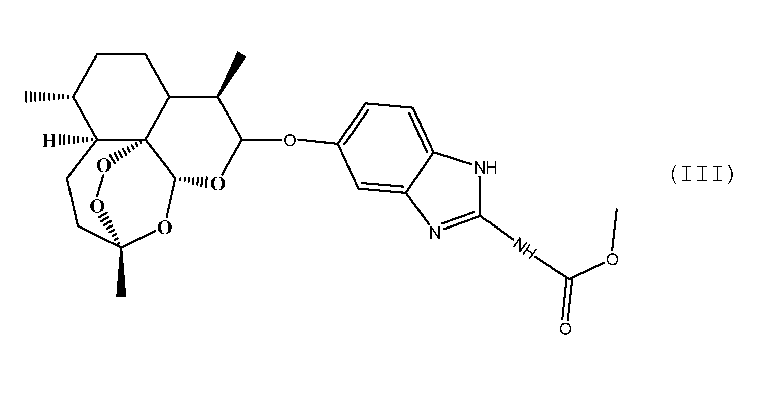

12. Pharmaceutical composition according to claim 1 or claim 2, wherein one or more artemisinin compounds are a compound according the formula (IV):

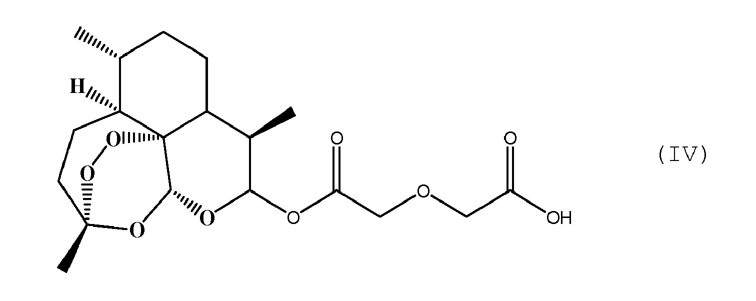

13. Pharmaceutical composition according to claim 1 or claim 2, wherein one or more artemisinin compounds are a compound according the formula (V):

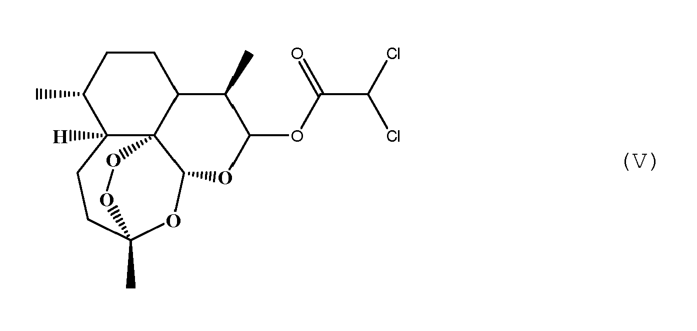

14. Pharmaceutical composition according to claim 1 or claim 2, wherein one or more artemisinin compounds are a compound according the formula (VI):

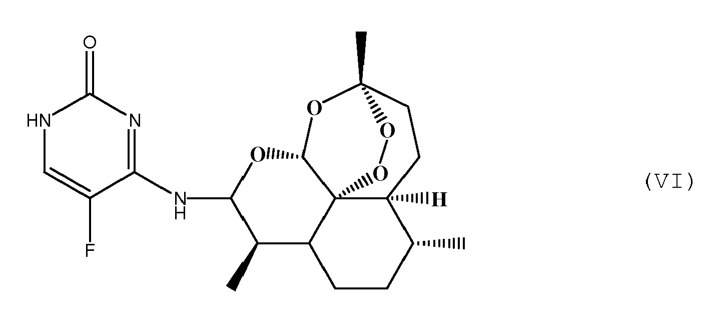

15. Pharmaceutical composition according to claim 1 or claim 2, wherein one or more artemisinin compounds are a compound according the formula (VII):

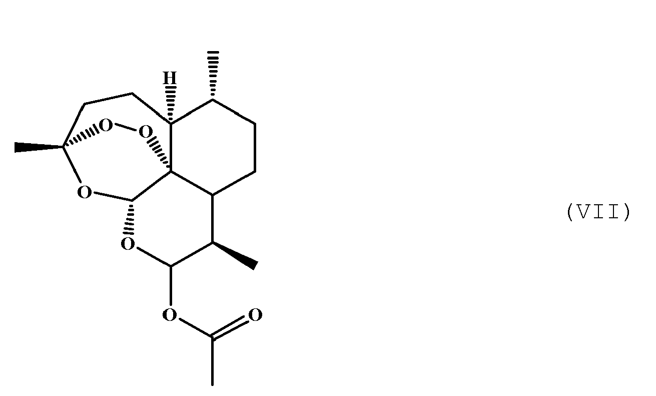

16. Pharmaceutical composition according to claim 1 or claim 2, wherein one or more artemisinin compounds are a compound according the formula (VIII):

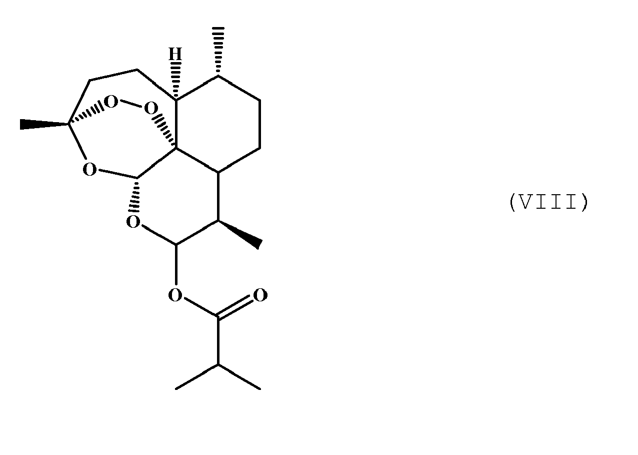

17. Pharmaceutical composition according to claim 1 or claim 2, wherein one or more artemisinin compounds are a compound according the formula (IX):

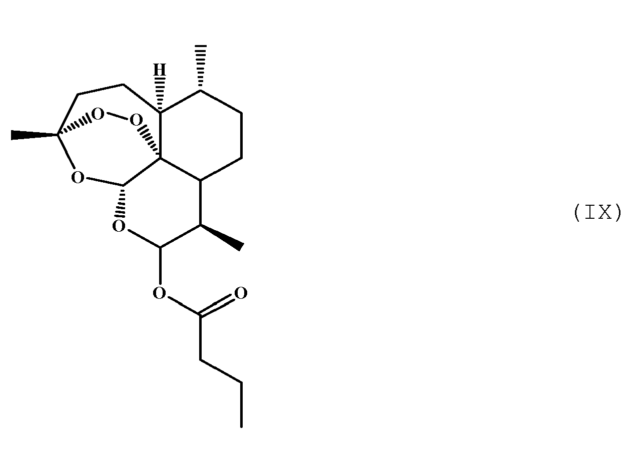

18. Pharmaceutical composition according to claim 1 or claim 2, wherein one or more artemisinin compounds are a compound according the formula (X):

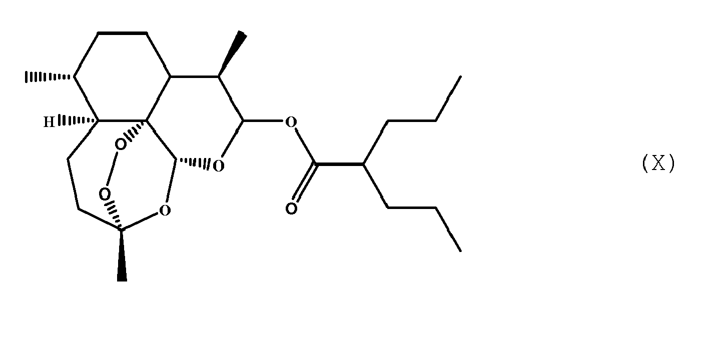

19. Pharmaceutical composition according to claim 1 or claim 2, wherein one or more artemisinin compounds are a compound according to the formula (XI):

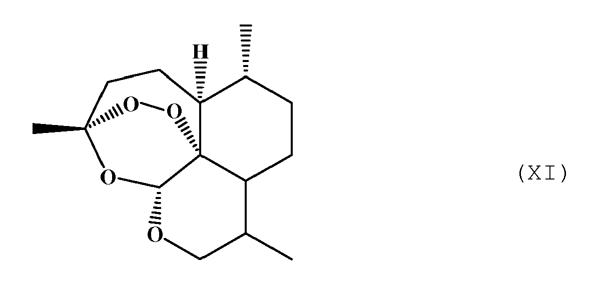

20. Pharmaceutical composition according to claim 1 or claim 2, wherein one or more artemisinin compounds are a compound according to the formula (XII):

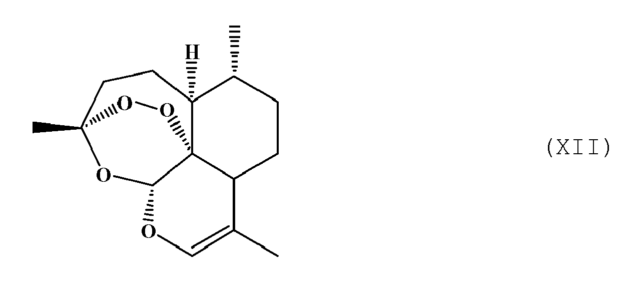

21. Pharmaceutical composition according to claim 1 or claim 2, wherein one or more artemisinin compounds are a compound according to the formula (XIII):

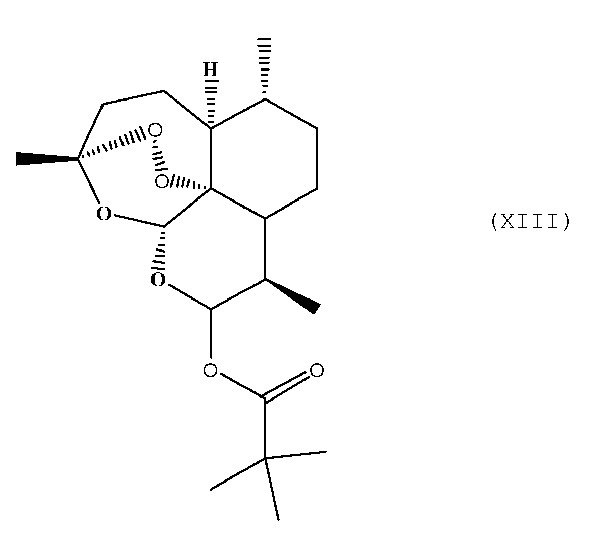

22. Pharmaceutical composition according to claim 1 or claim 2, wherein one or more artemisinin compounds are a compound according to the formula (XIV):

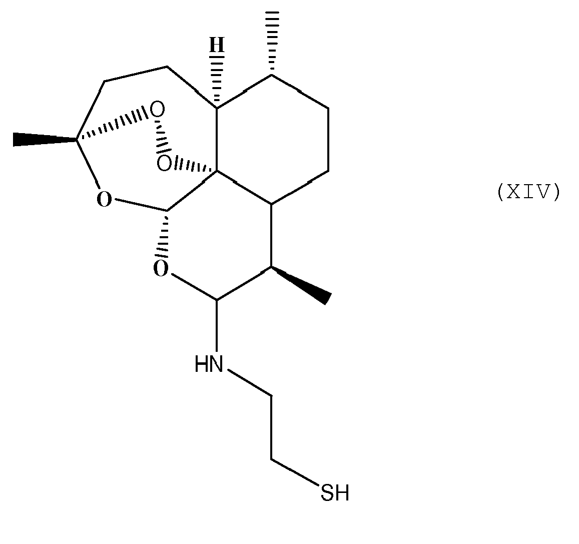

23. Pharmaceutical composition according to claim 1 or claim 2, wherein one or more artemisinin compounds are a compound according to the formula (XV):

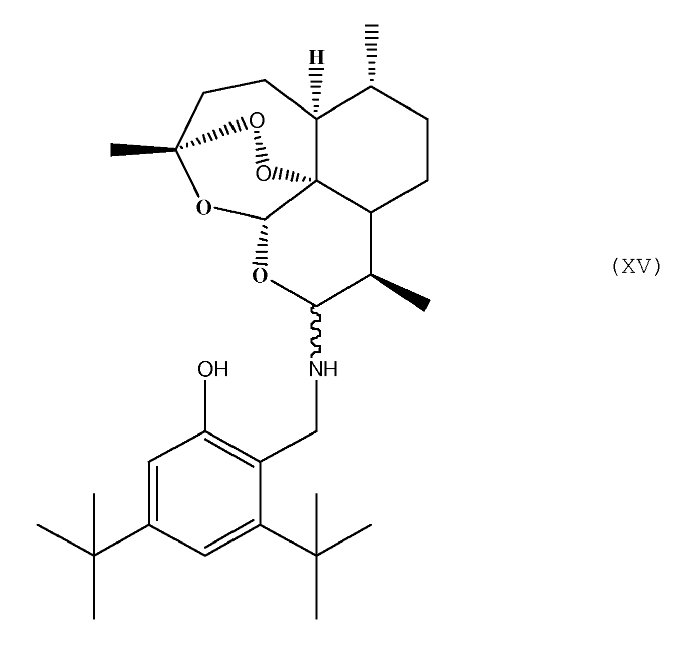

24. Pharmaceutical composition according to claim 1 or claim 2, wherein one or more artemisinin compounds are a compound according to the formula (XVI):

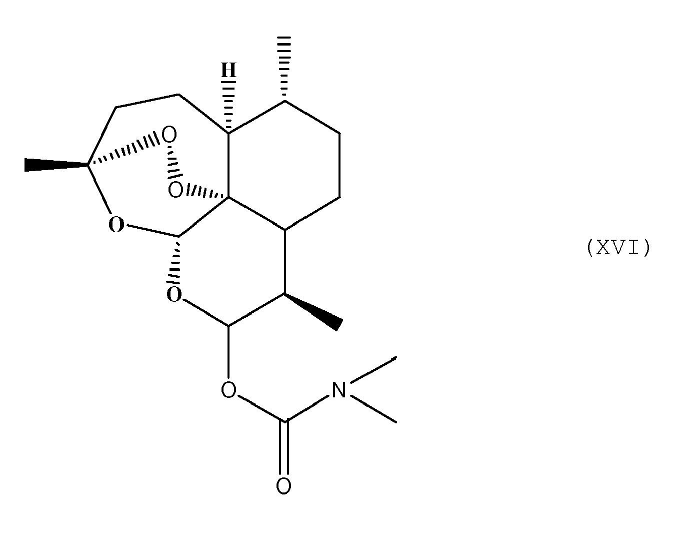

25. Pharmaceutical composition according to any of the claims 1 to 24, wherein said cancer is chosen from the group consisting of carcinoma, sarcoma, germ cell, blastoma and hematological.

26. Pharmaceutical composition according to claim 25, wherein said cancer is a hematological malignancy.

27. Pharmaceutical composition according to claim 26, wherein said hematological malignancy is chosen from the group consisting of lymphomas, acute lymphoblastic leukemia, myeloma, acute and chronic myelogenous leukemia, myelodysplastic syndromes and myeloproliferative diseases.

28. Pharmaceutical composition according to any of the claims 1 to 27, wherein said cancer is multiple myeloma.

29. Use of a pharmaceutical composition as defined in any of the claims 1 to 28 for the treatment of a cancer as defined in any of the claim 1 to 28.

30. Use of thalidomide, or a derivative thereof, as defined in any of the claims 1 to 28 and one or more artemisinin compounds as defined in any of the claims 1 to 28 for the treatment of a cancer as defined in any of the claims 1 to 28.
